# EUROPEAN PATENT SPECIFICATION

(11) **EP 0 638 072 B1**
(45) Date of publication and mention of the grant of the patent: **02.05.1997**
(21) Application number: 91904523.7
(22) Date of filing: 14.02.1991
(51) Int. Cl.: C07D 233/86, G01N 33/68

(54) **N,N-DISUBSTITUTED AMIC ACIDS AND THEIR AMMONIUM SALTS, AND THEIR USES THEREOF AS EMULSIFIERS**
N,N-DISUBSTITUIERTE AMIDSÄUREN, IHRE AMMONIUMSALZE SOWIE VERWENDUNG ALS EMULGATOR
ACIDES AMIQUES N,N-DISUBSTITUES ET LEURS SELS D'AMMONIUM AINSI QUE L'UTILISATION DE CEUX-CI COMME AGENTS EMULSIONNANTS

(30) Priority: 27.06.1990 US 542780
(43) Date of publication of application: 15.02.1995
(73) Proprietor: STEPAN COMPANY, Northfield, IL 60093 (US)
(72) Inventor: GOZE, Jean, M., Mundelein, IL 60060 (US); BERNHARDT, Randal, J., Antioch, IL 60002 (US); SAJIC, Branko, Chicago, IL 60625 (US); ROCKWELL, Ned, Miles, Lake Bluff, IL 60044 (US); McCONNELL, Nina, Montserrat, Sycamore, IL 60178 (US); MOHRING, William, Richard, Cresthill, IL 60435 (US)
(74) Representative: Cockbain, Julian, Dr.
(86) International application number: PCT/US91/00890
(87) International publication number: WO 92/00283

(56) References cited:
- EP-A- 0 126 483
- WO-A-91/01970
- US-A- 2 101 323
- RESEARCH DISCLOSURE, no. 310, February 1990, (New York, US), B. SAJIC: "Conditioning and antidandruff shampoo compositions with amidocarboxy benzoic acids and amidocarboxy benzoates as emulsifiers and suspending agents", pages 158-161.
- CHEMICAL ABSTRACTS, vol. 114, no. 1, 7 January 1991, (Columbus, Ohio, US), Y.J. UANG et al.: "W/O microemulsion studies with mono- and dialkyl amic acid surfactants", see page 99, abstract no. 8572f, & JOURNAL: J. COLLOID INTERFACE SCI. 1990, 139(2), 381-91.

## Description

### BACKGROUND OF THE INVENTION

This application is a continuation-in-part of pending U.S. Patent Application Serial No. 07/542,780 filed June 27, 1990.

### Field of the Invention

This invention relates to the use of N,N-disubstituted amic acids and/or N,N-disubstituted amic acid ammonium salts, their use as surfactants, emulsifiers, suspending agents and conditioning agents in shampoos.

### Description of the Related Art

This prior art is replete with emulsions for a myriad of uses. Among the numerous applications for emulsions are fabric softeners, surface cleaners, agricultural chemical mixtures, skin creams and lotions, car wax formulations, textile lubricants, and antiperspirant formulations.

These emulsions are the type wherein a liquid is dispersed in at least one insoluble liquid. Such emulsions include oil-in-water, water-in-oil, oil-in-water-in-a second oil, water-in-oil-in-a second oil, and oil-in-a second oil emulsions. These emulsions typically include oils such as crude petroleum oil, distilled petroleum oil, heavy paraffinic oil, asphaltene oil, linseed oil, tall oil, soybean oil alkyd, linseed oil alkyd, mineral oil, petrolatum, isopropyl palmitate, isopropyl myristate, caprylic/capric triglyceride, lanolin, acetylated lanolin alcohol, silicone compounds such as dimethicone and cyclomethicone, hydrogenated vegetable oil, sesame oil, safflower oil, avocado oil, glycerine, propylene glycol, sorbitol, C₁₂-C₁₆ alcohol benzoates, cocoa butter, vitamin E acetate, squalene, sodium pyrolidone carboxylic acid, methyl glucose ether, panthenol, melanin, and mixtures thereof.

Exemplary emulsions are shampoo and conditioner compositions. The prior art is also replete with shampoo and conditioner emulsions. Such emulsions are widely used because human hair becomes soiled due to its contact with the surrounding atmosphere and, to a greater extent, from sebum secreted by the head. The build-up of the sebum causes the hair to have a dirty feel and an unattractive appearance. The soiling of the hair necessitates its being shampooed with frequent regularity.

Shampooing the hair cleans by removing excess soil and sebum. However, the shampooing process is disadvantageous because the process results in hair that is left in a wet, tangled and generally unmanageable state. A variety of approaches have been developed to alleviate the after-shampoo problems. These range from the inclusion of hair conditioning aids in shampoos to post-shampoo application of hair conditioners, i.e., hair rinses. Hair rinses typically work by depositing a polymeric film or other material onto the hair. However, the use of such solutions as conditioners has not been fully satisfactory. For one thing, hair rinses are generally liquid in nature and must be applied in a separate step following the shampooing, left on the hair for a length of time, and finally rinsed with fresh water. This, of course, is time consuming and inconvenient. Furthermore, hair rinses or the leave-on hair conditioners, in addition to requiring an extra step, are difficult to apply in just the right amount of product and are not evenly distributed throughout a head of hair.

While shampoos have been disclosed which contain conditioning aids, these shampoos have not been totally satisfactory for a variety of reasons. One reason relates to a lack of compatibility between surfactants which are good cleaning agents and fatty cationic agents which are good conditioning agents. This lack of compatibility caused other surfactants such as nonionics, amphoterics and zwitterionics to be examined by workers in the field. However, no satisfactory solutions have been found. Post shampoo conditioners must be applied in a separate step after shampooing, left on the hair for a period of time and then rinsed with water. This process is time consuming and inconvenient.

Suspending/emulsifying agents have been used for cationics in shampoo compositions with surfactants and silicone materials. Normally, a suspension system comprising of xanthum gum, glycerol distearate and cetyl alcohol is used. Manufacturing these compositions is extremely complex, costly and time consuming.

U.S. Patent 4,741,855 describes shampoo compositions which comprise a synthetic surfactant, an insoluble, non-volatile silicone, a suspending agent, and water. The described suspending agents include long chain esters of ethylene glycol, esters of long chain fatty amine oxides and many others. There appear to be several key conditioning components in these compositions, including an insoluble, non-volatile, silicone, a suspending agent and a quaternary ammonium compound. The quaternary ammonium compounds, disclosed in U.S. Patent No. 4,741,855 as ingredients in a shampoo composition are di(hydrogenated tallow) dimethyl ammonium chloride and cetyltrimethyl ammonium chloride.

The use of silicone material in shampoos has been described in a number of different publications. The manufacture of such compositions is extremely complicated and requires specialized mixing equipment, high shear pumps, a heat exchanger, several manufacturing tanks, etc.

European patent application No. 89312074.1 teaches hair care compositions comprising from about 0.05% to about 10.0% of a nonrigid silicone gum. Dispersed in the gum is from about 0.01% to about 8.0% of an unsolubilized particulate matter which is preferably an octylacrylamide/acrylate/butylaminoethyl methacrylate copolymer.

Quaternary ammonium compounds derived from fatty acid amines such as tallow amine and di-tallow amine have been used as conditioners, surfactants and thickeners or emulsifiers in various shampoo and hair care products. For example, European Patent Application No. 0067635A2 discloses conditioning shampoos containing quaternary ammonium compounds of the formula: 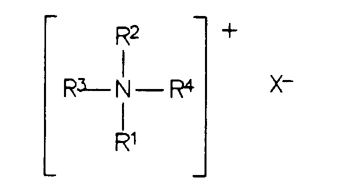 wherein the R¹ and R² groups contain an average of from about 16 to 22 carbon atoms, most preferably from about 16 to about 18 carbon atoms, R³ and R⁴ are C₁ to C₄ alkyl or hydroxyalkyl groups, and X is any compatible anion, particularly one selected from the group consisting of halide, hydroxide, methylsulfate, or acetate anions.

The shampoo compositions of that patent application also contain acyl derivatives which are long chain amides, alkanolamides, esters of ethylene glycol and glycerine, esters of carboxylic acids, esters of thiodicarboxylic acids, and mixtures of these derivatives. The shampoo compositions of that patent application also contain surfactants which are represented by the formula: 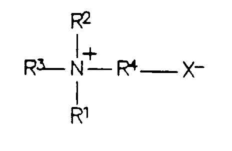 wherein R¹ is a long chain alkyl radical having from about 10 to about 18 carbon atoms or an amido radical represented by the formula: 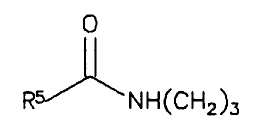 wherein R⁵ is a long chain alkyl radical, R² and R³ are each alkyl radicals having from about 1 to about 3 carbon atoms, R⁴ is an alkylene or hydroxy alkylene radical having from about 1 to about 4 carbon atoms, and X is a carboxylate radical.

European Patent Application No. 0 152 194 A2 discloses shampoo compositions containing quaternary ammonium salts of the formula: 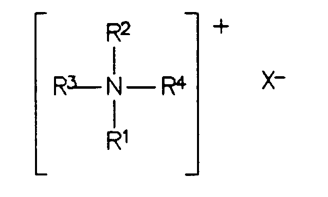 wherein R₁ is hydrogen, or an aliphatic group of from 1 to 22 carbon atoms, or an aromatic, aryl or alkaryl group having 6 to 20 carbon atoms; R₂ is an aliphatic group having from 12 to 22 carbon atoms; R₃ and R₄ are each alkyl groups having from 1 to 3 carbon atoms; and X is an anion selected from halogen, acetate, phosphate, nitrate and methyl sulfate radicals.

U.K. Patent Application No. GB 2196979 A provides hair care compositions comprising compounds of the formula: 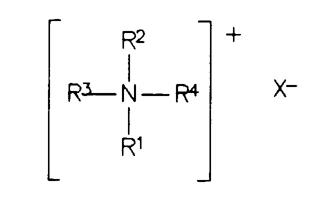 wherein R₁ and R₂ are aliphatic groups containing from about 12 to about 22 carbon atoms, R₃ and R₄ are hydrogen or short chain alkyl groups containing from about 1 to about 4 carbon atoms and X is anion selected from halogen, acetate, phosphate, nitrate and alkyl sulfate radicals.

U.K. Patent Application No. GB 2124647 A teaches quaternary ammonium compounds useful in shampoo compositions. The ammonium compounds have the formula: 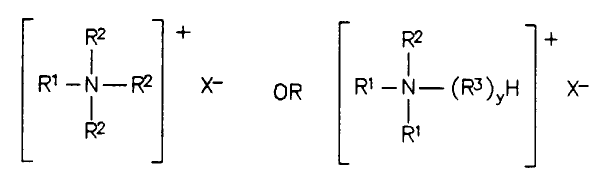 wherein R₁ is an aliphatic alkyl group containing an average of from about 16 to 22 carbon atoms, most preferably from about 16 to about 18 carbon atoms, the R₂ groups are C₁ to C₄ alkyl or hydroxylalkyl groups, the R₃ groups are alkylene oxide groups, preferably propylene oxide, where y is 1-4 and X is any compatible anion, particularly one selected from the group consisting of halide, hydroxide, methylsulfate, or acetate anions.

European Patent Application No. 0294 894 discloses conditioning agents for delivery from shampoos comprising compounds of the formula: 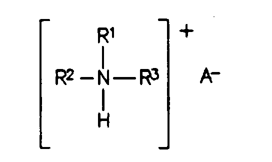 wherein R₁ and R₂ can independently be C₁₆ to C₂₀ alkyl or alkenyl and R³ is H or CH₃, and A is an anionic surfactant selected from the group consisting of alkyl sulfonates, aryl sulfonates, alkylaryl sulfonates, alkyl sulfates, alkyl ethoxylated sulfates, dialkyl sulfosuccinates, ethoxylated alkyl sulfonates, alkyl oxybenzene sulfonates, acyl isethionates, acyl alkyl taurates, olefin sulfonates and paraffin sulfonates.

A wide variety of surface active (surfactant) compounds are known and widely used. Further, certain relatively specific phthalamate derivatives are at least nominally disclosed in academic and patent literature. Some specific phthalamate derivatives have been suggested as being useful in plant growth regulator formulations, insect repellent formulations, bactericidal, fungicidal, herbicidal formulations, additives for improving low temperature flow characteristics of petroleum distillate fuels, solvent extraction formulations for certain heavy metal ions, catalyst systems for polyurethane foam formulations, additives for thermal recording materials, thickeners for silicone grease and oil-based drilling muds, additives for water-insensitive coatings, plasticizers, etc. Phthalamic acids or phthalamate derivatives have been used as additives for insecticidal compositions; additives for vulcanization activators; additives for rust and corrosion inhibitor formulations; additives to screen-clogging prevention and rust inhibition formulations; additives for improving low temperature flow characteristics of petroleum fuel oils; additives to catalyst systems for polyurethane foam formulations.

Ammonium phthalamates have been used as additives in fuel oil compositions, blending agents for grease, lubricating oil additives, and thickening agents for lubricating oil compositions. These ammonium phthalamates have the formula 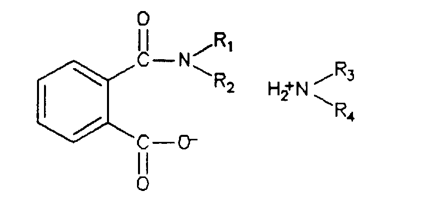 wherein
R₁, R₂, R₃ and R₄ are the C₁₆-C₄₀, preferably C₁₆-C₂₄ straight chain alkyl groups of secondary amine, and may be the same or different.

U.S. Patent No. 2,101,323 discloses compounds of the formula 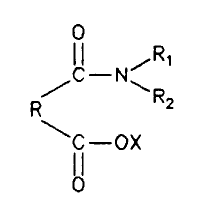 in which X is hydrogen or an alkali or alkaline earth metal, R represents the residue of a dicarboxylic acid, preferably an ortho-aromatic radical of the benzene series, R₁ is an aliphatic hydrocarbon radical containing eight or more carbon atoms and R₂ is hydrogen or anorganic radical, and which may be used in water-resistant, low-vapor pressure plasticizers for cellulose derivatives, and as wetting and detergent agents.

U.S. Patent No. 2,892,778 teaches grease compositions comprising metal soaps of terephthalic acid, terephthalamic acid, isophthalamic acid, and amic acids.

U.S. Patent No. 2,0915,464 teaches hydrocarbon lubricating oils comprising salts N-alkylamidophthalic acids where the N-alkyl substituent contains 8 to 18 carbon atoms.

U.S. Patent No. 2,971,027 discloses the use of diamides of terephthalic acid as grease thickening agents. These compounds have the general formula 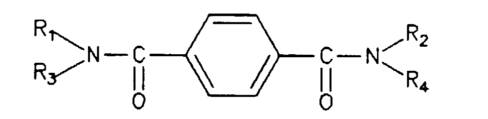 where R₁ and R₂ are selected from the group consisting of hydrocarbon and substituted hydrocarbon radicals attached to the nitrogen atoms by carbon-nitrogen bonds, and wherein R₃ and R₄ are selected from the group consisting of hydrogen, hydrocarbon and substituted hydrocarbon radicals, such hydrocarbon or substituted hydrocarbon radicals being attached to the nitrogen atoms by carbon-nitrogen bonds.

U.S. Patent No. 3,095,286 discloses compounds of the general formula 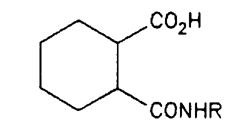 where R is a monovalent aliphatic hydrocarbon radical having between about 4 and 30 carbon atoms as being useful as fuel oil sediment inhibitors, anti-rust agents, and anti-screen clogging agents.

U.S. Patent No. 3,166,387 teaches pour point depressors for fuel oil compositions comprising salts of a secondary or tertiary monoamine having the formula: 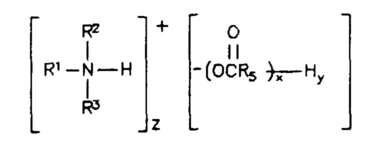 where R₁ and R₂ are alkyl groups containing from about 10 to about 22 carbon atoms, and R₃ and R₄ are hydrogen or alkyl groups containing from one to about 22 carbon atoms, x and z are each integers of from 1-4 and y is an integer of from 0 to 1.

U.S. Patent Nos. 4,333,082 and 3,544,467 disclose pour point depressants for hydrocarbon fuels and oils comprising succinamic acids of the formula 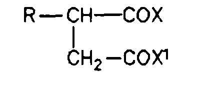 wherein R is a straight chain aliphatic hydrocarbon group having from 0 to 1 site of olefinic unsaturation (alkyl or alkenyl) attached at a secondary carbon atom to the succinyl group and is of at least 14 carbon atoms, generally in the range of 15 to 28 carbon atoms and more usually in the range of 15 to 22 carbon atoms. One of X and X¹ is hydroxyl and the other is:

-NYY¹

wherein N has its normal meaning of nitrogen and Y and Y¹ are aliphatic hydrocarbyl groups of from 14 to 28 carbon atoms, more usually of from 15 to 22 carbon atoms, having a total of from about 30 to 52 carbon atoms, more usually of from 32 to 48 carbon atoms, and, preferably, of from 32 to 40 carbon atoms, and the salts thereof.

U.S. Patent No. 3,658,453 teaches the use of various limited solubility wax crystal modifying amides or salts formed from acids with amines or ammonia to improve the cold flow properties of distillate fuel oils. Among the salts disclosed are various ammonium salts of fatty acids.

U.S. Patent No. 3,846,481 discloses di-(n-octadecyl) ammonium salts of aromatic carboxylic acids and their use as pour point depressants in hydrocarbon oils.

U.S. Patent No. 3,887,754 teaches the use of phthalic acid derivatives of the formula 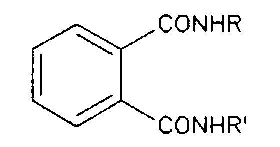 where R and R¹ are aliphatic hydrocarbon radicals of from 8 to 22 carbon atoms as coating substances for foamed polystyrene articles.

U.S. Patent No. 3,982,909 describes the use of various amides, diamides and ammonium salts of monoamides or monoesters of dibasic acids as wax crystal modifiers and cold flow improves for distillate fuel oils.

U.S. Patent Nos. 4,210,424 and 4,211,534 teach the case of oil-soluble combinations of (A) ethylene polymer of copolymer, (B) normal paraffinic wax composed of normal hydrocarbons whose average molecular weight is within the range of from 300 to 650, and (C) nitrogen compounds, such as amides, amine salts and ammonium salts, of carboxylic acids or anhydrides for use in improving the cold flow properties of distillate hydrocarbon fuel oils.

Journal of Colloid and Interface Science, 139: 381 (1990) describes the use of compounds of the formula 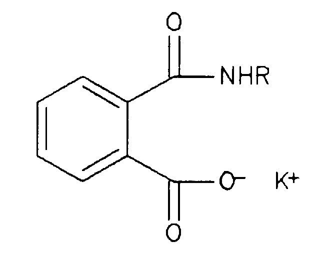 where R is octyl, dodecyl or oleyl as surfactants which stabilize water-in-oil microemulsions.

Tallow is a fatty acid byproduct of the meat-packing industry obtained by rendering the body fat from cattle and sheep. Tallows from different sources vary in free fatty acid content. The fatty acids normally found in tallow are myristic acid, palmitic acid, stearic acid, oleic acid, and linoleic acid.

Several methods are known for the preparation of tallow amines, but the most common method in industry is the conversion of a fatty acid to a nitrile by treating with ammonia, followed by catalytic hydrogenation of the nitrile to primary, secondary, or tertiary amine by suitable adjustment in the reaction conditions. Tallow amines, as well as di(hydrogenated tallow) amine, are commercially available; for example, di(hydrogenated tallow) amines are available under the trade name ARMEEN 2HT^{™} (Akzo Chemicals, Chicago, Illinois).

Various routes exist for the preparation of phthalamic acids and phthalamic acid salts. In U.S. Patent No. 4,402,708 N,N-diarachidyl phthalamic acid was prepared by adding phthalic anhydride to a 40% solution of amine in toluene in a 1/1 mole ratio at 80°C. The product was recovered by vacuum drying at 50°C, 0.05 mmHg for 20.5 hours. Phthalic anhydride sublimation was observed. This method makes no mention of the presence of any ammonium salt in the resultant product.

U.S. Patent No. 4,402,708 describes a method for preparing N,N-dioctadecyl phthalamic acid dioctadecyl ammonium salt and N,N-diarachidyl phthalamic acid diarachidyl ammonium salt. Phthalic anhydride was added to a 10% solution of amine in toluene in an anhydride to amine mole ratio of 1/2. The product was recovered by filtering and film evaporating a 1/1 toluene/n-heptane solution at 55°C, 40 mmHg.

Phthalamic acids have also been prepared by melting phthalic anhydride at 131°C and subsequent addition of molten secondary amine. The reactants to be added in an equimolar ratio. At the temperature used in this method, 131°C, excessive phthalic anhydride sublimation occurs and increased product degradation is observed. This method makes no mention of the presence of any ammonium salt in the resultant product.

Phthalamic acids have been prepared by addition of a solution of secondary amine in isopropanol at 78°C to a phthalic anhydride/isopropanol slurry at 78°C in a one to one phthalic anhydride/amine molar ratio with subsequent vacuum stripping of the solvent. This method utilizes isopropanol as the solvent for the reaction. Isopropanol, a secondary alcohol, reacts with phthalic anhydride to yield isopropyl mono ester of phthalic acid. At 78°C, as much as 40% of the product may be this ester.

Each of these methods produces a mixture of the desired phthalamic acid and an ammonium phthalamate salt. However, these methods make no mention of the presence of any ammonium salt in the resultant product.

### Summary of the Invention

It has been discovered that when certain N,N-disubstituted amic acids are incorporated into multiphasic mixtures and emulsified, the resulting emulsions exhibit excellent stability. These amic acids unexpectedly function as emulsifying agents for a variety of multiphasic emulsified products. Among these emulsion products are shampoos, conditioners, skin creams and lotions, agricultural chemical mixtures, fabric softeners, dishwashing detergents, antiperspirants, liquid soaps and surface cleaners. These N,N-disubstituted amic acids also behave as suspending agents for particles that are insoluble in a liquid system. For example, these amic acids may be employed as suspending agents in suspensions such as anti-dandruff shampoos, antiperspirants and deodorant formulations.

Viewed from one aspect the present invention provides an emulsion characterised in that it contains an effective emulsifying amount of a compound of formula I: 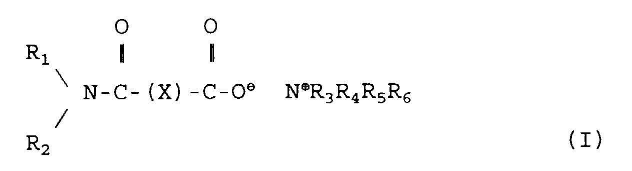 wherein R₁ and R₂ are the same or different and represent alkyl or alkenyl having 8 to 40 carbon atoms, R₃ and R₄ are the same as R₁ and R₂, and R₅ and R₆ are hydrogen; or R₃, R₄, R₅, and R₆ are independently hydrogen, alkyl or alkenyl having 1 to 40 carbon atoms when R₁ and R₂ are alkyl or alkenyl having 14-22 carbon atoms; and X represents ortho, para, or meta substituted phenyl, or alkylene or alkenylene having 0-6 carbon atoms, or cycloalkylene having 5 to 7 carbon atoms.

Viewed from a further aspect, the present invention provides a formulated shampoo composition characterised in that it contains an amount in the range of about 0.05% to about 20% of a salt of formula I as defined above.

Viewed from a still further aspect the invention provides a suspension of particles in a liquid system comprising particles that are insoluble in the liquid system characterised in that said suspension contains an effective suspending amount of a salt of formula I as defined above.

Viewed from a yet further aspect the invention provides an antiperspirant formulation characterised in that it contains an amount in the range of about 0.05% to about 20% of a salt of formula I as defined above.

The various compositions according to the invention may further comprise an acid of formula II: 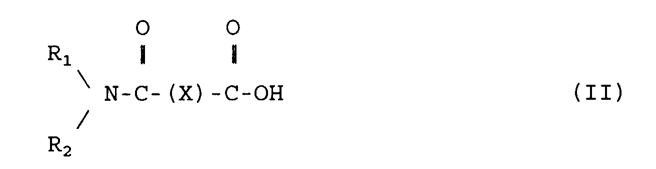 wherein R₁ and R₂ are the same or different and represent alkyl or alkenyl having 8 to 40 carbon atoms, and X represents ortho, para, or meta substituted phenyl, or alkylene or alkenylene having 2-6 carbon atoms, or cycloalkylene having 5 to 7 carbon atoms.

### DETAILED DESCRIPTION OF THE INVENTION

It has been discovered that when certain N,N-disubstituted amic acids are incorporated into multiphasic mixtures and emulsified, the resulting emulsions exhibit excellent stability. These amic acids unexpectedly function as emulsifying agents for a variety of multiphasic emulsified products. Among these product emulsions are shampoos, conditioners, skin creams and lotions, agricultural chemical mixtures, fabric softeners, dishwashing detergents, antiperspirants, liquid soaps, and surface cleaners. These N,N-disubstituted amic acids also behave as suspending agents for particles that are insoluble in a liquid system. For example, these amic acids may be employed as suspending agents in suspensions such as anti-dandruff shampoos, antiperspirants and deodorant formulations.

Accordingly, the present invention encompasses emulsions and suspensions comprising compounds of formula I 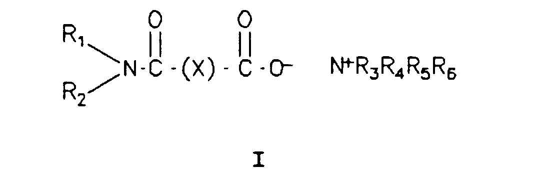 wherein R₁ and R₂ are the same or different and represent alkyl or alkenyl having 8 to 40 carbon atoms, R₃ and R₄ are the same as R₁ and R₂, and R₅ and R₆ are hydrogen; or R₃, R₄, R₅, and R₆ are independently hydrogen, alkyl or alkenyl having 1 to 40 carbon atoms when R₁ and R₂ are alkyl or alkenyl having 14-22 carbon atoms; and X represents ortho, para, or meta substituted phenyl, or alkylene or alkenylene having 0-6 carbon atoms, or cycloalkylene having 5 to 7 carbon atoms.

By X in formula I is meant an ortho, meta, or para substituted phenyl group, or methylene, ethylene, propylene, butylene, pentylene, hexylene, etheneylene, propenylene, 1-, or 2- butenylene, 1-, 2- or 3- pentylene, or 1-, 2-or 3-hexenylene, cyclohexylene or cyclohexenylene. The diacids resulting from the variation of X groups in Formula I include phthalic, isophthalic, terephthalic, oxalic, malonic, glutaric, adipic, pimelic, suberic, maleic, succinic, 1,2-cyclohexanoic, 1,3-cyclohexanoic and, 1,4 cyclohexanoic acids.

The emulsions of the invention are the type wherein a liquid is dispersed in at least one insoluble liquid. Those skilled in the art will recognize a variety of emulsions where the compounds of the present invention may be used. The emulsions of the invention include emulsions such as, for example, oil-in-water, water-in-oil, oil-in-water-in-a second oil, water-in-oil-in-a second oil, and oil-in-a second oil emulsions.

The oils which may be employed in the emulsions of the present invention include oils such as, for example, crude petroleum oil, distilled petroleum oil, heavy paraffinic oil, asphaltene oil, linseed oil, tall oil, soybean oil alkyd, linseed oil alkyd, mineral oil, petrolatum, isopropyl palmitate, isopropyl myristate, caprylic/capric triglyceride, lanolin, acetylated lanolin alcohol, silicone compounds such as dimethicone and cyclomethicone, hydrogenated vegetable oil, sesame oil, safflower oil, avocado oil, glycerine, propylene glycol, sorbitol, C₁₂-C₁₆ alcohol benzoates, cocoa butter, vitamin E acetate, squalene, sodium pyrolidone carboxylic acid, methyl glucose ether, panthenol and melanin. Mixtures of these oils may also be used in the emulsions of the invention.

The silicone compounds which may be used in the emulsions of the present invention are well known and have been generally taught to be useful in a variety of emulsions. Generally such silicone compounds may be represented by the formula: 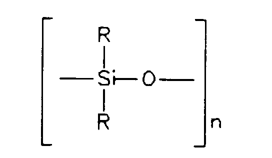 wherein R is a 1 to 3 carbon alkyl group, n is a number from 3 to 10, preferably from 3 to 7, and the unsatisfied valences on the oxygen and silicon atoms at the ends of the chain may be joined to one another to form a cyclic structure. Suitable silicone compounds are, for example, U.C.C. Y-7207, sold by Union Carbide Corporation in which each R is methyl and which typically comprises by weight 99.4% tetramer, 0.6% trimer and traces of the pentamer and hexamer; SWS-03314, sold by SWS Silicones, a Division of Stauffer Chemical Company, in which R is methyl and which is substantially all tetramer; and Dow Corning 344 fluid, sold by Dow Corning, Inc., in which R is methyl and which typically comprises by weight about 88% tetramer, about 11.8% pentamer and traces of trimer and hexamer. Typical vapor pressures of silicones are shown below. These vapor pressures were determined using Dow Corning 344 fluid at various temperatures.

| Temperature | Vapor Pressure, mmHg |
|---|---|
| 26°C | 1 |
| 64°C | 10 |
| 77°C | 20 |
| 92°C | 40 |
| 101°C | 60 |
| 114°C | 100 |
| 155°C | 400 |
| 178°C | 760 |

By suspension is meant a mixture of particles or solid particulate matter evenly distributed throughout a liquid system in which the particles are insoluble. The particles may be of any composition, i.e., they may be organic or inorganic compounds. In addition, various organometallic compounds, such as the solid, insoluble, active antiperspirant aluminum and zirconium salts, may be suspended by the amic acids and salts of the invention. These aluminum and zirconium salts include aluminum chloride, aluminum chlorhydroxide, basic aluminum bromide, zirconyl chloride, zirconyl hydroxide, complexes of aluminum hydroxide, zirconyl chloride and aluminum chlorhydroxide, complexes of aluminum hydroxide, zirconyl hydroxychloride and aluminum chlorhydroxide, complexes of dihydroxyaluminum glycinate, zirconyl chloride and/or zirconyl hydroxychloride and aluminum chlorhydroxides, complexes of zirconyl chloride and/or zirconyl hydroxychloride with aluminum chlorhydroxide and an amino acid, such as glycine (as a buffering agent). The liquid systems may be aqueous, monoaqueous, or an emulsion as described above.

The effective concentration of these N,N-disubstituted amic acid ammonium salts in the emulsions of the present invention varies from about 0.05% to about 20% on an active basis. A preferred use concentration appears to be between about 0.5% to 10%. A more preferred concentration is from about 3% to about 7%.

The N,N-disubstituted amic acids ammonium salts of the invention are particularly well suited for use in emulsions having water and at least one insoluble liquid. The insoluble liquid is typically an oil or a combination of oils. Such an emulsion may be termed an aqueous emulsion.

The emulsions and suspensions of the invention may further comprise an acid of formula II: 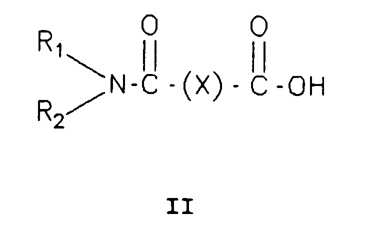 wherein R₁ and R₂ are the same or different and represent alkyl or alkenyl having 8 to 40 carbon atoms, and X represents ortho, para, or meta substituted phenyl, or alkylene or alkenylene having 2-6 carbon atoms, or cycloalkylene having 5 to 7 carbon atoms.

By X in formula I is meant an ortho, meta, or para substituted phenyl group, or methylene, ethylene, propylene, butylene, pentylene, hexylene, etheneylene, propenylene, 1-, or 2- butenylene, 1-, 2- or 3- pentylene, or 1-, 2-or 3-hexenylene, cyclohexylene or cyclohexenylene. The diacids resulting from the variation of X groups in Formula I include phthalic, isophthalic, terephthalic, oxalic, malonic, glutaric, adipic, pimelic, suberic, maleic, succinic, 1,2-cyclohexanoic, 1,3-cyclohexanoic and, 1,4 cyclohexanoic acids.

The emulsions and suspensions of the invention comprising a mixture of a salt of formula I and an acid of formula II have ratios of acid to salt varying from about 99:1 to about 1:99. Preferred ratios of acid to salt vary from about 70:30 to about 30:70.

The effective concentration of mixtures of the N,N-disubstituted amic acids and their ammonium salts in the emulsions and suspensions of the present invention varies from about 0.05% to about 20% on an active basis. A presently preferred use concentration appears to be between about 0.5% to 10%. A more preferred concentration is between 3% and 7%.

The emulsions of the present invention are readily manufactured using any conventional emulsification process. The manufacture of the emulsions may be effected using either single-phase hot processes or multi-phase processes.

The N,N-disubstituted amic acids and their ammonium salts are particularly well-suited as emulsifiers for non-volatile and volatile silicones and other water insoluble emollient oils. The utilization of this technology can be naturally extended to car wax formulations, textile lubricants, textile anti-static agents, shoe polishes, antiperspirants, hair conditioners and shampoos and to other uses of silicone products.

In a preferred embodiment of the emulsions and suspensions of the invention, R₁, R₂, R₃, and R₄, are derived from hydrogenated tallow and R₅ and R₆ are hydrogen. Because tallow is a mixture of C-14 to C-18 fatty acids, and amines derived from tallow are a mixture of tallow amines, the amic acids and/or the ammonium salts thereof used in the present invention may, therefore, have R groups that are the same or different. Particularly preferred amic acids and ammonium salts according to the present invention include di(hydrogenated) tallow ammonium di(hydrogenated) tallow succinamate, di(hydrogenated) tallow succinamic acid, di(hydrogenated) tallow ammonium di(hydrogenated) tallow cyclohexamate, di(hydrogenated) tallow cyclohexamic acid, di(hydrogenated) tallow ammonium di(hydrogenated) isophthalamate, di(hydrogenated) isophthalamic acid, di(hydrogenated) tallow ammonium di(hydrogenated) tallow maleamate, di(hydrogenated) tallow maleamic acid, di(hydrogenated) tallow ammonium di(hydrogenated) tallow oxalamate, di(hydrogenated) tallow oxalamic acid, di(hydrogenated) tallow ammonium di(hydrogenated) tallow malonamate, di(hydrogenated) tallow malonamic acid, di(hydrogenated) tallow ammonium di(hydrogenated) tallow glutaramate, di(hydrogenated) tallow glutaramic acid, di(hydrogenated) tallow ammonium di(hydrogenated) tallow adipamate, di(hydrogenated) tallow adipamic acid, di(hydrogenated) tallow ammonium di(hydrogenated) tallow pimelamate, di(hydrogenated) tallow pimelamic acid, di(hydrogenated) tallow ammonium di(hydrogenated) tallow subaramate, and di(hydrogenated) tallow subaramic acid.

In certain embodiments of the invention the emulsion is a formulated shampoo composition which comprises an effective emulsifying amount of an N,N-disubstituted amic acid ammonium salt of formula I. It has been discovered that when certain amic acid ammonium salts are incorporated into a typical shampoo base with a non-volatile, insoluble silicone and the resulting formulated shampoo product is evaluated in a laboratory setting on hair swatches or in a salon setting on human subjects, perceptual improvements in detangling, wet combability, dry combability, and static control are readily observed. These compounds unexpectedly function as emulsifying/suspending agents for silicone compounds, as anti-static or conditioning agents, and as surfactants.

The effective concentration of these amic acid ammonium salts in the shampoos of the present invention varies from about 0.05% to about 20% on an active basis. A presently preferred use concentration appears to be between about 0.5% to 10%. More preferred concentrations are from about 3% to about 7%.

The shampoo compositions of the invention may further comprise an acid of formula II.

In a preferred embodiment of the shampoo compositions of the invention, R₁, R₂, R₃, and R₄, are derived from hydrogenated tallow and R₅ and R₆ are hydrogen. Because tallow is a mixture of C-14 to C-18 fatty acids, and amines derived from tallow are a mixture of tallow amines, the amic acids and/or the ammonium salts thereof used in the present invention may, therefore, have R groups that are the same or different. Particularly preferred amic acids and ammonium salts according to the present invention include di(hydrogenated) tallow ammonium di(hydrogenated) tallow succinamate, di(hydrogenated) tallow succinamic acid, di(hydrogenated) tallow ammonium di(hydrogenated) tallow cyclohexamate, di(hydrogenated) tallow cyclohexamic acid, di(hydrogenated) tallow ammonium di (hydrogenated) isophthalamate, and di(hydrogenated) isophthalamic acid, di(hydrogenated) tallow ammonium de(hydrogenated) tallow maleamate, and di(hydrogenated) tallow maleamic acid, di(hydrogenated) tallow ammonium di(hydrogenated) tallow oxalamate, di(hydrogenated) tallow oxalamic acid, di(hydrogenated) tallow ammonium di(hydrogenated) tallow malonamate, di(hydrogenated) tallow malonamic acid, di(hydrogenated) tallow ammonium di(hydrogenated) tallow glutaramate, di(hydrogenated) tallow glutaramic acid, di(hydrogenated) tallow ammonium di(hydrogenated) tallow adipamate, di(hydrogenated) tallow adipamic acid, di(hydrogenated) tallow ammonium di(hydrogenated) tallow pimelamate, di(hydrogenated) tallow pimelamic acid, di(hydrogenated) tallow ammonium di(hydrogenated) tallow subaramate, and di(hydrogenated) tallow subaramic acid.

The shampoo compositions of the invention comprising a mixture of a salt of formula I and an acid of formula II have ratios of acid to salt varying from about 99:1 to about 10:99. Preferred ratios of acid to salt vary from about 70:30 to about 30:70.

The effective concentration of mixtures of the N,N-disubstituted amic acids and their ammonium salts in the shampoos of the present invention varies from about 0.05% to about 20% on an active basis. A presently preferred use concentration appears to be between about 0.5% to 10%. A more preferred concentration is between 3% and 7%.

The laboratory and salon results indicate that certain amic acid ammonium salts, acids and/or mixtures thereof, may have a dual function in this type of application. The shampoo formulations prepared according to the present invention with certain amic acid, amic acid ammonium salts, or mixtures thereof, exhibit excellent flow properties and have long term stability at different storage temperatures. The finished formulations retain all of the above properties after at least three freeze-thaw cycles and are easily preserved using common preservatives.

The shampoos of the present invention are readily manufactured using a conventional single-phase, hot process. The manufacture of the conditioning shampoos using a single-phase, hot process is simple and, therefore, preferred over multi-phase processes.

The shampoos of the present invention are prepared by incorporating salts of formula I and or acids of formula II in typical shampoo bases. The shampoo formulations and emulsions of the invention may also include pearling agents, emollients, humectants, proteins, amino acids, and polymers. Those skilled in the art will recognize that desired properties may be attained by adding certain ingredients to these formulations. A representative list of such ingredients may be found in the Cosmetic, Toiletry and Fragrance Association (CTFA) Dictionary.

The invention also provides antiperspirant emulsions and suspensions comprising an effective emulsifying/ suspending amount of an N,N-disubstitued amic acid ammonium salt of of formula I.

The effective concentration of these amic acid ammonium salts in the antiperspirants of the present invention varies from about 0.05% to about 20% on an active basis. A presently preferred use concentration appears to be between about 0.5% to 10%. More preferred concentrations are from about 3% to about 7%.

The antiperspirant compositions of the invention may further comprise an acid of formula II.

In a preferred embodiment of the antiperspirant compositions of the invention, R₁, R₂, R₃, and R₄, are derived from hydrogenated tallow and R₅ and R₆ are hydrogen. Because tallow is a mixture of C-14 to C-18 fatty acids, and amines derived from tallow are a mixture of tallow amines, the amic acids and/or the ammonium salts thereof used in the present invention may, therefore, have R groups that are the same or different.

Particularly preferred amic acids and ammonium salts according to the present invention include di(hydrogenated) tallow ammonium di(hydrogenated) tallow succinamate, di(hydrogenated) tallow succinamic acid, di(hydrogenated) tallow ammonium di(hydrogenated) tallow cyclohexamate, di(hydrogenated) tallow cyclohexamic acid, di(hydrogenated) tallow ammonium di(hydrogenated) isophthalamate, and di(hydrogenated) isophthalamic acid, di(hydrogenated) tallow ammonium de(hydrogenated) tallow maleamate, and di(hydrogenated) tallow maleamic acid, di(hydrogenated) tallow ammonium di(hydrogenated) tallow oxalamate, di(hydrogenated) tallow oxalamic acid, di(hydrogenated) tallow ammonium di(hydrogenated) tallow malonamate, di(hydrogenated) tallow malonamic acid, di(hydrogenated) tallow ammonium di(hydrogenated) tallow glutaramate, di(hydrogenated) tallow glutaramic acid, di(hydrogenated) tallow ammonium di(hydrogenated) tallow adipamate, di(hydrogenated) tallow adipamic acid, di(hydrogenated) tallow ammonium di(hydrogenated) tallow pimelamate, di(hydrogenated) tallow pimelamic acid, di(hydrogenated) tallow ammonium di(hydrogenated) tallow subaramate, and di(hydrogenated) tallow subaramic acid.

The antiperspirant compositions of the invention comprising a mixture of a salt of formula I and an acid of formula II have ratios of acid to salt varying from about 99:1 to about 1:99. Preferred ratios of acid to salt vary from about 70:30 to about 30:70.

The effective concentration of mixtures of the N,N-disubstituted amic acids and their ammonium salts in the antiperspirants of the present invention varies from about 0.05% to about 20% on an active basis. A presently preferred use concentration appears to be between about 0.5% to 10%. A more preferred concentration is between 3% and 7%.

The antiperspirant formulations of the present invention may be anhydrous or water-in-oil formulations and may be in the form of roll-on, stick or spray-on products. The amic acids and/or amic acid salts of the invention may be employed in any type of antiperspirant formulation.

The antiperspirant formulations of the invention will also include at least one active antiperspirant salt. Preferred antiperspirant salts for use in the invention are aluminum and/or zirconium salts. Suitable materials for use in both anhydrous and water-in-oil formulations are, for example, aluminum chloride, aluminum chlorhydroxide, basic aluminum bromide, zirconyl chloride, zirconyl hydroxide, complexes of aluminum hydroxide, zirconyl chloride and aluminum chlorhydroxide, complexes of aluminum hydroxide, zirconyl hydroxychloride and aluminum chlorhydroxide, complexes of dihydroxyaluminum glycinate, zirconyl chloride and/or zirconyl hydroxychloride and aluminum chlorhydroxides, complexes of zirconyl chloride and/or zirconyl hydroxychloride with aluminum chlorhydroxide and an amino acid, such as glycine (as a buffering agent), and mixtures of two or more of the above. The antiperspirant used is generally soluble in water, but insoluble in the silicone material, which is hydrophobic. The amount of antiperspirant present may be varied to suit particular needs. In general, the formulations will comprise from about 2 to 30% antiperspirant salt by weight, and preferably from about 10 to 25% antiperspirant salt. There must be enough of the active antiperspirant material present for the formulation to be effective as an antiperspirant. On the other hand it is expected that concentrations above about 20-30% of antiperspirant salt may be outside present regulatory limits. (As used herein the antiperspirant salt concentrations are based upon equivalent amounts of the particular hydrated salt). A particularly effective antiperspirant salt, designated according to its Cosmetic, Toiletry and Fragrance Association (CTFA) adopted name, is aluminum zirconium tetrachlorohydrex glycine, a coordination complex of aluminum zirconium tetrachlorohydrate and glycine, in which some of the water molecules normally coordinated to the metals have been displaced by glycine. These materials are available commercially, for example from Dow Corning, Inc. under the designation Dow Corning AZG 368, 369, 370, or 374.

The antiperspirants of the invention may further include montmorillonite clays. The montmorillonite clays that may be used in the anhydrous formulations of the present invention include montmorillonite clays such as Bentone 38 (known in CTFA momenclature as Quaternium-18 Hectorite) and Bentone 34 (known in CTFA nomenclature as Quaternium-18 Bentonite) as well as other suitable clays.

The present invention also encompasses methods for preparing mixtures of N,N-disubstituted amic acids and their corresponding ammonium salts.

The invention is illustrated further by the following examples which are not to be construed as limiting the invention in scope or spirit to the specific procedures described in them.

### EXAMPLE 1

### Preparation of a mixture of N,N-di(hydrogenated tallow) amic acid and N,N-di(hydrogenated tallow) amic acid di-(hydrogenated tallow) ammonium salt

One mole (148.0g) of flaked phthalic anhydride (PA) was charged into a 4-neck 5-liter round bottomed flask reactor equipped with a mechanical stirrer, a thermocouple temperature controller and a heating mantle. A charge of 650g of isopropyl alcohol (USP grade) was added to the reactor to achieve a slurry of about 50% solids. One mole (about 502g) of molten di(hydrogenated tallow) amine was slowly added to the slurry in the reactor with continuous stirring. The temperature of the reaction mass was allowed to stabilize at about 45-55°C with gradual addition of the amine and cooling of the reactor flask. Amine addition was completed in about 0.5 to 1 hours. Thereafter the reactor was maintained at about 60°C until the PA flakes dissipated and IR spectroscopic analysis showed no detectable amounts of PA in the reaction mass. Isopropyl alcohol was then removed under vacuum (about 1-50 mmHg) On analysis, about 62 mole % acid and about 38 mole % salt were found with an average molecular weight of about 782.

### EXAMPLE 2

Maleic anhydride (0.25 mol) was added to a flask and heated to about 80°C. To the liquid maleic anhydride was added 0.338 mol of liquid di(hydrogenated) tallow amine. The resulting mixture was stirred for 150 minutes at 80°C and, after workup, yielded a mixture of di(hydrogenated) tallow ammonium di(hydrogenated) tallow maleamate and di(hydrogenated) tallow maleamic acid.

### EXAMPLE 3

Succinic anhydride (0.25 mol) was added to a flask and melted by heating to about 120°C. Liquid di(hydrogenated) tallow amine (0.33 mol) was then added to the anhydride with stirring over 10 minutes. This mixture was stirred for 2 hours at 120°C and, after workup yielded a mixture of di(hydrogenated) tallow ammonium di(hydrogenated) tallow succinamate and di(hydrogenated) tallow succinamic acid.

### EXAMPLE 4

Di(hydrogenated) tallow amine (1.35 mol) was melted in a flask at about 70°C. To the melted amine was added hexahydrophthalic anhydride (1 mol) with stirring at about 70-75°C.

The resulting mixture was then stirred at about 70°C for 4 hours, and, upon workup, yielded a mixture of di(hydrogenated) tallow ammonium di(hydrogenated) tallow cyclohexamate and di(hydrogenated) tallow cyclohexamic acid.

### EXAMPLE 5

Dimethylterephthalate (0.25 mol) was melted in a flask at about 145°C. To the melted terephthate was then added 0.25 mol of liquid di(hydrogenated) tallow amine. The mixture was stirred at about 145°C for 4 hours, followed by an additional 4 hours at about 230°C. The mixture was coded and esterified by treating with 1 M NaOH (0.13 mol) at 100°C for 6 hours. The sodium salt was converted to di(hydrogenated) tallow ammonium di(hydrogenated) tallow terephthalamate by the addition of di(hydrogenated) tallow ammonium hydrochloride to yield a mixture of the acid and salt.

### EXAMPLE 6

Dimethyl isophthalate (0.50 mol) was melted in a flask at about 70°C. To the melted isophthalate was added 0.25 mol of liquid di(hydrogenated) tallow amine with stirring. The reaction mixture was stirred at about 135-140°C for 6 hours. To this mixture was then added 1 M NaOH (0.25 mol) and the resulting mixture stirred at 100°C for 6 additional hours. The sodium salt was treated with di(hydrogenated) tallow ammonium hydrochloride to yield a mixture of di(hydrogenated) tallow ammonium di(hydrogenated) tallow isophthalamate and di(hydrogenated) tallow isophthalamic acid.

### EXAMPLE 7

### Preparation of a Conditioning Shampoo

Water was first added into a suitable vessel equipped with agitation, heating and cooling capabilities. While the water was agitated and heated slowly, tetrasodium EDTA, STEPANOL^{®} AM-V (ammonium lauryl sulfate) and NINOL^{®} 40 CO (cocomide diethanolamine) were added. At about 145°F a 70:30 mixture of N, N-di(hydrogenated tallow) phthalamic acid and N,N-di(hydrogenated tallow) phthalamic acid N,N-di(hydrogenated tallow) ammonium salt, followed by silicone DC 200 (dimethicone) were added to the mixture. The mixture was heated to 160-165°F and emulsified for 20-30 minutes at high speed while maintaining the temperature between 160-165°F. The mixture was slowly cooled with agitation set at medium speed. When the temperature of the mixture cooled to about 110°C, 1,3,5,5-tetramethyl hydantoin and ammonium chloride were added. The pH was checked and adjusted as necessary with ammonium hydroxide or citric acid to a value between about 4.5 to 6.5. The viscosity was checked and adjusted as necessary with ammonium chloride to a value between 2000 and 6000 cps. (Formulation 1)

### EXAMPLE 8

Conditioning shampoos were prepared with different amic acid/ammonium salt mixtures essentially according to Example 7 to yield the following formulations.

Formulation 2 was prepared to contain a mixture of di(hydrogenated) tallow ammonium di(hydrogenated) tallow maleamate and di(hydrogenated) tallow maleamic acid.

Formulation 3 was prepared to contain a mixture of di(hydrogenated) tallow ammonium di(hydrogenated) tallow succinamate and di(hydrogenated) tallow succinamic acid.

Formulation 4 was prepared to contain a mixture of di(hydrogenated) tallow ammonium di(hydrogenated) tallow cyclohexamate and di(hydrogenated) tallow cyclohexamic acid.

Formulation 5 was prepared to contain a mixture of di(hydrogenated) tallow ammonium di(hydrogenated) tallow terephthalamate and di (hydrogenated) tallow terephthalamic acid.

Formulation 6 was prepared to contain a mixture of di(hydrogenated) tallow ammonium di(hydrogenated) tallow isophthalamate and di(hydrogenated) tallow isophthalamic acid.

Formulation 7 was prepared to contain a mixture of dicoco ammonium dicoco phthalamate and dicoco phthalamic acid.

### EXAMPLE 9

### Preparation of an anti-dandruff shampoo

Water was first added into a suitable vessel equipped with agitation, heating and cooling capabilities. While the water was agitated and heated slowly, tetrasodium EDTA, STEPANOL^{®} AM-V, (ammonium lauryl sulfate) and NINOL^{®} 40 CO, (cocomide diethanolamine) were added. At about 145°F, a 70:30 mixture of N, N-di(hydrogenated tallow) phthalamic acid and N,N-di(hydrogenated tallow) phthalamic acid N,N-di(hydrogenated tallow) ammonium salt, followed by silicone DC 200 (dimethicone) and ZPT (zinc pyrithione, 48% dispersion), were added to the mixture. The mixture was heated to 160-165°F and emulsified for 20-30 minutes at high speed while maintaining the temperature between 160-165°F. The mixture was slowly cooled with agitation set at medium speed. When the temperature of the mixture cooled to about 110°C, 1,3,5,5-tetramethyl hydantoin and ammonium chloride were added. The pH was checked and adjusted as necessary with ammonium hydroxide or citric acid to a value between about 4.5 and 6.5. The viscosity was checked and adjusted as necessary with ammonium chloride to a value between 2000 and 6000 cps. (Formulation 8)

### EXAMPLE 10

Anti-dandruff shampoos were prepared with different amic acid/ammonium salt mixtures essentially according to Example 9 to yield the following formulations.

Formulation 9 was prepared to contain a mixture of di(hydrogenated) tallow ammonium di(hydrogenated) tallow maleamate and di(hydrogenated) tallow maleamic acid.

Formulation 10 was prepared to contain a mixture of di(hydrogenated) tallow ammonium di(hydrogenated) tallow succinamate and di(hydrogenated) tallow succinamic acid.

Formulation 11 was prepared to contain a mixture of di(hydrogenated) tallow ammonium di(hydrogenated) tallow cyclohexamate and di(hydrogenated) tallow cyclohexamic acid.

Formulation 12 was prepared to contain a mixture of di (hydrogenated) tallow ammonium di(hydrogenated) tallow terephthalamate and di(hydrogenated) tallow terephthalamic acid.

Formulation 13 was prepared to contain a mixture of di(hydrogenated) tallow ammonium di(hydrogenated) tallow isophthalamate and di(hydrogenated) tallow isophthalamic acid.

Formulation 14 was prepared to contain a mixture of dicoco ammonium dicoco phthalamate and dicoco phthalamic acid.

### EXAMPLE 11

### Preparation of an Anti-dandruff/Conditioning Shampoo

Water was first added into a suitable vessel equipped with agitation, heating and cooling capabilities. While the water was agitated and heated slowly, tetrasodium EDTA, STEPANOL^{®} AM-V, (ammonium lauryl sulfate), and NINOL^{®} 40 CO (cocomide diethanolamine) were added. At about 145°F, a 70:30 mixture of N, N-di(hydrogenated tallow) phthalamic acid and N,N-di(hydrogenated tallow) phthalamic acid N,N-di(hydrogenated tallow) ammonium salt, followed by silicone DC 200 (dimethicone) and ZPT (zinc pyrithione, 48% dispersion) were added to the mixture. The mixture was heated to 160-165°F and emulsified for 20-30 minutes at high speed while maintaining the temperature between 160-165°F. The mixture was slowly cooled with agitation set at medium speed. When the temperature of the mixture cooled to about 110°C, 1,3,5,5-tetramethyl hydantoin and ammonium chloride were added. The pH was checked and adjusted as necessary with ammonium hydroxide or citric acid to a value between about 4.5 and 6.5. The viscosity was checked and adjusted as necessary with ammonium chloride to a value between 2000 and 6000 cps. (Formulation 15)

### EXAMPLE 12

Anti-dandruff/conditioning shampoos were prepared with different amic acid/ammonium salt mixtures essentially according to Example 11 to yield the following formulations.

Formulation 16 was prepared to contain a mixture of di(hydrogenated) tallow ammonium di(hydrogenated) tallow maleamate and di(hydrogenated) tallow maleamic acid.

Formulation 17 was prepared to contain a mixture of di(hydrogenated) tallow ammonium di(hydrogenated) tallow succinamate and di(hydrogenated) tallow succinamic acid.

Formulation 18 was prepared to contain a mixture of di(hydrogenated) tallow ammonium di(hydrogenated) tallow cyclohexamate and di(hydrogenated) tallow cyclohexamic acid.

Formulation 19 was prepared to contain a mixture of di(hydrogenated) tallow ammonium di(hydrogenated) tallow terephthalamate and di (hydrogenated) tallow terephthalamic acid.

Formulation 20 was prepared to contain a mixture of di(hydrogenated) tallow ammonium di(hydrogenated) tallow isophthalamate and di(hydrogenated) tallow isophthalamic acid.

Formulation 21 was prepared to contain a mixture of dicoco ammonium dicoco phthalamate and dicoco phthalamic acid.

As will be appreciated, the above formulations provide exemplary primary emulsifiers only; other secondary emulsifiers, amides and mixtures thereof may be used.

The formulations prepared according to Examples 7-12 were evaluated in shampoo screening tests. The results of this evaluation are depicted in Table 1 as follows.

**TABLE 1**

| Shampoo Formulation Evaluation | | | |
|---|---|---|---|
| Formulation Number | Emulsion Quality | Performance on Hair Swatches | Stability Room Temp.to 110°F |
| 1 | High | Excellent | Stable |
| 2 | High | Excellent | Stable |
| 3 | High | Excellent | Stable |
| 4 | High | Excellent | Stable |
| 5 | High | Excellent | Stable |
| 6 | High | Excellent | Stable |
| 7 | Low | Poor | Unstable |
| 8 | High | Excellent | Stable |
| 9 | High | Excellent | Stable |
| 10 | High | Excellent | Stable |
| 11 | High | Excellent | Stable |
| 12 | High | Excellent | Stable |
| 13 | High | Excellent | Stable |
| 14 | Low | Poor | Unstable |
| 15 | High | Excellent | Stable |
| 16 | High | Excellent | Stable |
| 17 | High | Excellent | Stable |
| 18 | High | Excellent | Stable |
| 19 | High | Excellent | Stable |
| 20 | High | Excellent | Stable |
| 21 | Low | Poor | Unstable |

### EXAMPLE 13

### Preparation of an Antiperspirant Formulation With an Amic Acid/Salt Mixture

Silicone 344 (Cyclomethicone) (70.0g) and di(hydrogenated) tallow ammonium di (hydrogenated) tallow maleamate and di(hydrogenated) tallow maleamic acid (10.0g) is added to a reaction vessel and heated to about 55-57°C. Dow Corning AZG 370 (AlZr tetrachlorohydrex glycine) (20g) is subsequently added and the mixture agitated for 15 minutes. This mixture is then cooled to about 32-35°C and subsequently homogenized. The resultant product is a stable, creamy antiperspirant formulation.

From the foregoing it will be appreciated that, although specific embodiments of the invention have been described herein for purposes of illustration, various modifications may be made.

## Claims

1. An emulsion characterised in that it contains an effective emulsifying amount of a salt of the formula 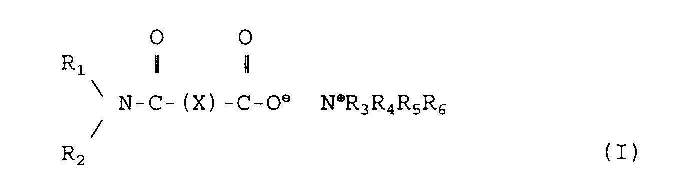 wherein R₁ and R₂ are the same or different and represent alkyl or alkenyl having 8 to 40 carbon atoms, R₃ and R₄ are the same as R₁ and R₂, and R₅ and R₆ are hydrogen; or R₃, R₄, R₅, and R₆ are independently hydrogen, alkyl or alkenyl having 1 to 40 carbon atoms when R₁ and R₂ are alkyl or alkenyl having 14-22 carbon atoms; and X represents ortho, para, or meta substituted phenyl, or alkylene or alkenylene having 1-6 carbon atoms, or cycloalkylene having 5 to 7 carbon atoms.

2. An emulsion according to claim 1, wherein the amount of the salt is from about 0.05% to about 20%.

3. An emulsion according to claim 2, wherein the amount of the salt is from about 0.5% to about 10%.

4. An emulsion according to claim 3, wherein R₁, R₂, R₃ and R₄ are fatty tallow alkyl groups, and R₅ and R₆ are hydrogen.

5. An emulsion according to claim 3, wherein the salt is selected from the group consisting of di(hydrogenated) tallow ammonium di(hydrogenated) tallow succinamate, di (hydrogenated) tallow ammonium di (hydrogenated) tallow cyclohexamate, di(hydrogenated) tallow ammonium di (hydrogenated) tallow isophthalamate, di(hydrogenated) tallow ammonium di(hydrogenated) tallow maleamate, di(hydrogenated) tallow ammonium di(hydrogenated) tallow oxalamate, di(hydrogenated) tallow ammonium di (hydrogenated) tallow malonamate, di (hydrogenated) tallow ammonium di(hydrogenated) tallow glutaramate, di(hydrogenated) tallow ammonium di(hydrogenated) tallow adipamate, di(hydrogenated) tallow ammonium di (hydrogenated) tallow pimelamate, and di (hydrogenated) tallow ammonium di(hydrogenated) tallow subaramate.

6. An emulsion according to claim 5, further comprising an acid of the formula 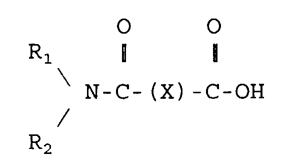 (wherein R₁ and R₂ are the same or different and represent alkyl or alkenyl having 8 to 40 carbon atoms, and X represents ortho, para, or meta substituted phenyl, or alkylene or alkenylene having 1-6 carbon atoms, or cycloalkylene having 5 to 7 carbon atoms) where the combined amount of the acid and salt is from about 0.05% to about 20%.

7. An emulsion according to claim 6, wherein the acid and salt are present in a ratio ranging from about 99:1 to about 1:99.

8. An emulsion according to claim 7, wherein the acid and salt are present in a ratio ranging from about 70:30 to about 30:70.

9. An emulsion according to claim 7, wherein the combined amount of the acid and the salt is from about 0.5% to about 10%.

10. An emulsion according to claim 9, wherein the combined amount of the acid and the salt is about 3-7%.

11. An emulsion according to claim 10, further comprising an effective conditioning amount of at least one silicone compound.

12. A formulated shampoo composition characterised in that it contains an amount in the range of about 0.05% to about 20% of a salt of the formula: 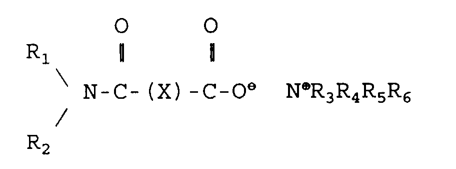 wherein R₁ and R₂ are the same or different and represent alkyl or alkenyl having 8 to 40 carbon atoms, R₃ and R₄ are the same as R₁ and R₂, and R₅ and R₆ are hydrogen; or R₃, R₄, R₅, and R₆ are independently hydrogen, alkyl or alkenyl having 1 to 40 carbon atoms when R₁ and R₂ are alkyl or alkenyl having 14-22 carbon atoms; and X represents ortho, para, or meta substituted phenyl, or alkylene or alkenylene having 1-6 carbon atoms, or cycloalkylene having 5 to 7 carbon atoms.

13. A shampoo composition according to claim 12, wherein the amount of the salt is from about 0.05% to about 20%.

14. A shampoo composition according to claim 13, wherein R₁, R₂, R₃ and R₄ are fatty tallow alkyl groups, and R₅ and R₆ are hydrogen.

15. A shampoo composition according to claim 14, further comprising an effective conditioning amount of at least one silicone compound.

16. A shampoo composition according to claim 13, further comprising an acid of the formula 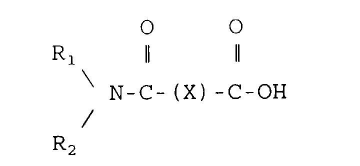 (wherein R₁ and R₂ are the same or different and represent alkyl or alkenyl having 8 to 40 carbon atoms, and X represents ortho, para, or meta substituted phenyl, or alkylene or alkenylene having 1-6 carbon atoms, or cycloalkylene having 5 to 7 carbon atoms) where the combined amount of the acid and salt is from about 0.05% to about 20%.

17. A shampoo composition according to claim 16, wherein the acid and salt are present in a ratio ranging from about 99:1 to about 1:99.

18. A shampoo composition according to claim 17, wherein the salt is selected from the group consisting of di(hydrogenated) tallow ammonium di(hydrogenated) tallow succinamate, di(hydrogenated) tallow ammonium di (hydrogenated) tallow cyclohexamate, di (hydrogenated) tallow ammonium di(hydrogenated) tallow isophthalamate, di (hydrogenated) tallow ammonium di (hydrogenated) tallow maleamate, di(hydrogenated) tallow ammonium di (hydrogenated) tallow oxalamate, di(hydrogenated) tallow ammonium di(hydrogenated) tallow malonamate, di (hydrogenated) tallow ammonium di (hydrogenated) tallow glutaramate, di(hydrogenated) tallow ammonium di (hydrogenated) tallow adipamate, di(hydrogenated) tallow ammonium di(hydrogenated) tallow pimelamate, and di(hydrogenated) tallow ammonium di(hydrogenated) tallow subaramate.

19. A suspension of particles in a liquid system comprising particles that are insoluble in the liquid system characterised in that said suspension contains an effective suspending amount of a salt of the formula: 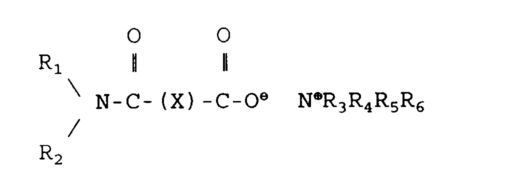 wherein R₁ and R₂ are the same or different and represent alkyl or alkenyl having 8 to 40 carbon atoms, R₃ and R₄ are the same as R₁ and R₂, and R₅ and R₆ are hydrogen; or R₃, R₄, R₅, and R₆ are independently hydrogen, alkyl or alkenyl having 1 to 40 carbon atoms when R₁ and R₂ are alkyl or alkenyl having 14-22 carbon atoms; and X represents ortho, para, or meta substituted phenyl, or alkylene or alkenylene having 1-6 carbon atoms, or cycloalkylene having 5 to 7 carbon atoms.

20. An antiperspirant formulation characterised in that it contains an amount in the range of about 0.05% to about 20% of a salt of the formula: 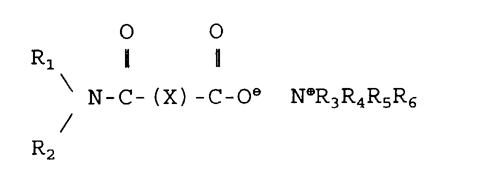 wherein R₁ and R₂ are the same or different and represent alkyl or alkenyl having 8 to 40 carbon atoms, R₃ and R₄ are the same as R₁ and R₂, and R₅ and R₆ are hydrogen; or R₃, R₄, R₅, and R₆ are independently hydrogen, alkyl or alkenyl having 1 to 40 carbon atoms when R₁ and R₂ are alkyl or alkenyl having 14-22 carbon atoms; and X represents ortho, para, or meta substituted phenyl, or alkylene or alkenylene having 1-6 carbon atoms, or cycloalkylene having 5 to 7 carbon atoms.

21. A formulation according to claim 20, wherein the amount of the salt is from about 0.05% to about 20%.

22. A formulation according to claim 21, wherein R₁, R₂, R₃ and R₄ are fatty tallow alkyl groups, and R₅ and R₆ are hydrogen.

23. A formulation according to claim 20, further comprising an acid of the formula 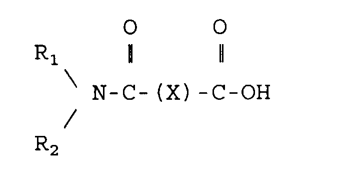 (wherein R₁ and R₂ are the same or different and represent alkyl or alkenyl having 8 to 40 carbon atoms, and X represents ortho, para, or meta substituted phenyl, or alkylene or alkenylene having 1-6 carbon atoms, or cycloalkylene having 5 to 7 carbon atoms) where the combined amount of the acid and salt is from about 0.05% to about 20%.

## Patentansprüche

1. Emulsion, dadurch gekennzeichnet, daß sie eine zur Bildung einer Emulsion wirksame Menge eines Salzes der Formel 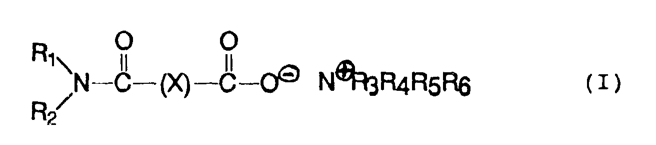 enthält,
worin R₁ und R₂ gleich oder verschieden sind und für Alkyl oder Alkenyl mit 8 bis 40 Kohlenstoffatomen stehen, R₃ und R₄ die gleichen Bedeutungen wie R₁ und R₂ besitzen und R₅ und R₆ für Wasserstoff stehen; oder R₃, R₄, R₅ und R₆ unabhängig voneinander für Wasserstoff, Alkyl oder Alkenyl mit 1 bis 40 Kohlenstoffatomen stehen, wenn R₁ und R₂ für Alkyl oder Alkenyl mit 14 - 22 Kohlenstoffatomen stehen; und X für ortho-, para- oder meta-substituiertes Phenyl oder Alkylen oder Alkenylen mit 1 - 6 Kohlenstoffatomen oder Cycloalkylen mit 5 bis 7 Kohlenstoffatomen steht.

2. Emulsion nach Anspruch 1, worin das Salz in einer Menge von etwa 0,05 % bis etwa 20 % vorhanden ist.

3. Emulsion nach Anspruch 2, worin das Salz in einer Menge von etwa 0,5 % bis etwa 10 % vorhanden ist.

4. Emulsion nach Anspruch 3, worin R₁, R₂, R₃ und R₄ für Fettalgalkylgruppen und R₅ und R₆ für Wasserstoff stehen.

5. Emulsion nach Anspruch 3, worin das Salz ausgewählt ist unter di(hydriertes)-Talgammonium-di(hydriertes)-Talgsuccinamat, di(hydriertes)-Talgammonium-di(hydriertes)-Talgcyclohexamat, di(hydriertes)-Talgammonium-di(hydriertes)-Talgisophthalamat, di(hydriertes)-Talgammonium-di(hydriertes)-Talgmaleamat, di(hydriertes)-Talgammonium-di(hydriertes)-Talgoxalamat, di(hydriertes)-Talgammonium-di(hydriertes)-Talgmalonamat, di(hydriertes)-Talgammonium-di(hydriertes)-Talgglutaramat, di(hydriertes)-Talgammonium-di(hydriertes)-Talgadipamat, di(hydriertes)-Talgammonium-di(hydriertes)-Talgpimelamat und di(hydriertes)-Talgammonium-di(hydriertes)-Talgsubaramat.

6. Emulsion nach Anspruch 5, weiter umfassend eine Säure der Formel 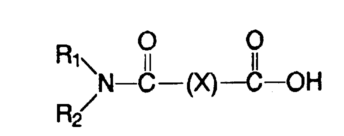 (worin R₁ und R₂ gleich oder verschieden sind und für Alkyl oder Alkenyl mit 8 bis 40 Kohlenstoffatomen stehen und X für ortho-, para- oder meta-substituiertes Phenyl oder Alkylen oder Alkenylen mit 1 - 6 Kohlenstoffatomen oder Cycloalkylen mit 5 bis 7 Kohlenstoffatomen steht), wobei Säure und Salz zusammen in einer Menge von etwa 0,05 % bis etwa 20 % vorhanden sind.

7. Emulsion nach Anspruch 6, worin Säure und Salz in einem Verhältnis im Bereich von etwa 99:1 bis etwa 1:99 vorhanden sind.

8. Emulsion nach Anspruch 7, worin Säure und Salz in einem Verhältnis im Bereich von etwa 70:30 bis etwa 30:70 vorhanden sind.

9. Emulsion nach Anspruch 7, worin Säure und Salz zusammen in einer Menge von etwa 0,5 % bis etwa 10 % vorhanden sind.

10. Emulsion nach Anspruch 9, worin Säure und Salz zusammen in einer Menge von etwa 3-7 % vorhanden sind.

11. Emulsion nach Anspruch 10, weiter umfassend eine konditionierend wirkende Menge wenigstens einer Silikonverbindung.

12. Formulierte Shampoo-Zusammensetzung, dadurch gekennzeichnet, daß sie ein Salz der Formel 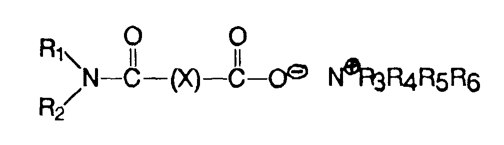 worin R₁ und R₂ gleich oder verschieden sind und für Alkyl oder Alkenyl mit 8 bis 40 Kohlenstoffatomen stehen, R₃ und R₄ die gleichen Bedeutungen wie R₁ und R₂ besitzen und R₅ und R₆ für Wasserstoff stehen; oder R₃, R₄, R₅ und R₆ unabhängig voneinander für Wasserstoff, Alkyl oder Alkenyl mit 1 bis 40 Kohlenstoffatomen stehen, wenn R₁ und R₂ für Alkyl oder Alkenyl mit 14 - 22 Kohlenstoffatomen stehen; und X für ortho-, para- oder meta-substituiertes Phenyl oder Alkylen oder Alkenylen mit 1 - 6 Kohlenstoffatomen oder Cycloalkylen mit 5 bis 7 Kohlenstoffatomen steht, in einer Menge im Bereich von etwa 0,05 % bis etwa 20 % enthält.

13. Shampoo-Zusammensetzung nach Anspruch 12, worin das Salz in einer Menge von etwa 0,05 % bis etwa 20 % vorhanden ist.

14. Shampoo-Zusammensetzung nach Anspruch 13, worin R₁, R₂, R₃ und R₄ für Fettalgalkylgruppen und R₅ und R₆ für Wasserstoff stehen.

15. Shampoo-Zusammensetzung nach Anspruch 14, weiter umfassend eine konditionierend wirkende Menge wenigstens einer Silikonverbindung.

16. Shampoo-Zusammensetzung nach Anspruch 13, weiter umfassend eine Säure der Formel 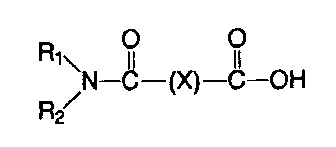 (worin R₁ und R₂ gleich oder verschieden sind und für Alkyl oder Alkenyl mit 8 bis 40 Kohlenstoffatomen stehen und X für ortho-, para- oder meta-substituiertes Phenyl oder Alkylen oder Alkenylen mit 1 - 6 Kohlenstoffatomen oder Cycloalkylen mit 5 bis 7 Kohlenstoffatomen steht), wobei Säure und Salz zusammen in einer Menge von etwa 0,05 % bis etwa 20 % vorhanden sind.

17. Shampoo-Zusammensetzung nach Anspruch 16, worin Säure und Salz in einem Verhältnis im Bereich von etwa 99:1 bis etwa 1:99 vorhanden sind.

18. Shampoo-Zusammensetzung nach Anspruch 17, worin das Salz ausgedwählt ist unter di(hydriertes)-Talgammonium-di(hydriertes)-Talgsuccinamat, di(hydriertes)-Talgammonium-di(hydriertes)-Talgcyclohexamat, di(hydriertes)-Talgammonium-di(hydriertes)-Talgisophthalamat, di(hydriertes)-Talgammonium-di(hydriertes)-Talgmaleamat, di(hydriertes)-Talgammonium-di(hydriertes)-Talgoxalamat, di(hydriertes)-Talgammonium-di(hydriertes)-Talgmalonamat, di(hydriertes)-Talgammonium-di(hydriertes)-Talgglutaramat, di(hydriertes)-Talgammonium-di(hydriertes)-Talgadipamat, di(hydriertes)-Talgammonium-di(hydriertes)-Talgpimelamat und di(hydriertes)-Talgammonium-di(hydriertes)-Talgsubaramat.

19. Suspension von Teilchen in einem flüssigen System, umfassend Teilchen, die in dem flüssigen System unlöslich sind, dadurch gekennzeichnet, daß die Suspension eine zur Bildung einer Suspension wirksame Menge eines Salzes der Formel: 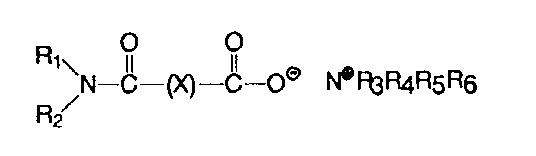 enthält,
worin R₁ und R₂ gleich oder verschieden sind und für Alkyl oder Alkenyl mit 8 bis 40 Kohlenstoffatomen stehen, R₃ und R₄ die gleichen Bedeutungen wie R₁ und R₂ besitzen und R₅ und R₆ für Wasserstoff stehen; oder R₃, R₄, R₅ und R₆ unabhängig voneinander für Wasserstoff, Alkyl oder Alkenyl mit 1 bis 40 Kohlenstoffatomen stehen, wenn R₁ und R₂ für Alkyl oder Alkenyl mit 14 - 22 Kohlenstoffatomen stehen; und X für ortho-, para- oder meta-substituiertes Phenyl oder Alkylen oder Alkenylen mit 1 - 6 Kohlenstoffatomen oder Cycloalkylen mit 5 bis 7 Kohlenstoffatomen steht.

20. Antiperspiransformulierung, dadurch gekennzeichnet, daß sie ein Salz der Formel 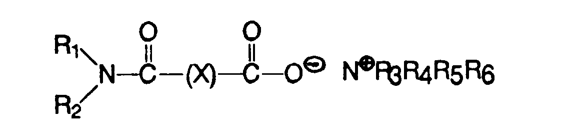 worin R₁ und R₂ gleich oder verschieden sind und für Alkyl oder Alkenyl mit 8 bis 40 Kohlenstoffatomen stehen, R₃ und R₄ die gleichen Bedeutungen wie R₁ und R₂ besitzen und R₅ und R₆ für Wasserstoff stehen; oder R₃, R₄, R₅ und R₆ unabhängig voneinander für Wasserstoff, Alkyl oder Alkenyl mit 1 bis 40 Kohlenstoffatomen stehen, wenn R₁ und R₂ für Alkyl oder Alkenyl mit 14 - 22 Kohlenstoffatomen stehen; und X für ortho-, para- oder meta-substituiertes Phenyl oder Alkylen oder Alkenylen mit 1 - 6 Kohlenstoffatomen oder Cycloalkylen mit 5 bis 7 Kohlenstoffatomen steht, in einer Menge im Bereich von etwa 0,05 % bis etwa 20 % enthält.

21. Formulierung nach Anspruch 20, worin das Salz in einer Menge von etwa 0,05 % bis etwa 20 % vorhanden ist.

22. Formulierung nach Anspruch 21, worin R₁, R₂, R₃ und R₄ für Fettalgalkylgruppen und R₅ und R₆ für Wasserstoff stehen.

23. Formulierung nach Anspruch 20, weiter umfassend eine Säure der Formel 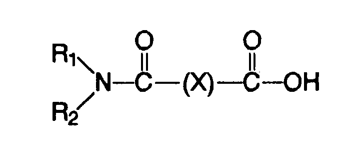 (worin R₁ und R₂ gleich oder verschieden sind und für Alkyl oder Alkenyl mit 8 bis 40 Kohlenstoffatomen stehen und X für ortho-, para- oder meta-substituiertes Phenyl oder Alkylen oder Alkenylen mit 1 - 6 Kohlenstoffatomen oder Cycloalkylen mit 5 bis 7 Kohlenstoffatomen steht), wobei Säure und Salz zusammen in einer Menge von etwa 0,05 % bis etwa 20 % vorhanden sind.

## Revendications

1. Emulsion caractérisée en ce qu'elle contient une quantité émulsionnante efficace d'un sel de formule 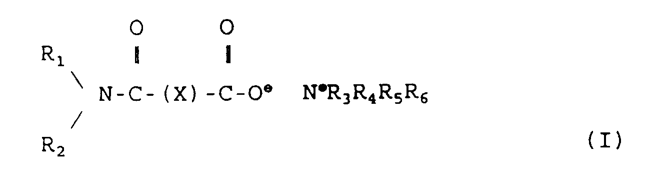 dans laquelle R₁ et R₂ sont identiques ou différents et représentent un alkyle ou un alcényle ayant de 8 à 40 atomes de carbone, R₃ et R₄ sont identiques à R₁ et R₂, et R₅ et R₆ sont un atome d'hydrogène ; ou R₃, R₄ , R₅ et R₆ sont, indépendamment, un atome d'hydrogène, un alkyle ou un alcényle ayant de 1 à 40 atomes de carbone quand R₁ et R₂ sont un alkyle ou un alcényle ayant de 14 à 22 atomes de carbone ; et X représente un phényle ortho, para, ou métasubstitué, ou un alkylène ou un alcénylène ayant de 1 à 6 atomes de carbone, ou un cycloalkylène ayant de 5 à 7 atomes de carbone.

2. Emulsion selon la revendication 1, dans laquelle la quantité de sel est d'environ 0,05 à environ 20%.

3. Emulsion selon la revendication 2, dans laquelle la quantité de sel est d'environ 0,5 à environ 10%.

4. Emulsion selon la revendication 3, dans laquelle R₁, R₂, R₃ et R₄ sont des groupes alkyles gras de suif, et R₅ et R₆ sont un atome d'hydrogène.

5. Emulsion selon la revendication 3, dans laquelle le sel est choisi dans le groupe formé par le succinamate di(hydrogéné) de suif d'ammonium di(hydrogéné) de suif, le cyclohexamate di(hydrogéné) de suif d'ammonium di(hydrogéné) de suif, l'isophtalamate di(hydrogéné) de suif d'ammonium di(hydrogéné) de suif, le maléamate di(hydrogéné) de suif d'ammonium di(hydrogéné) de suif, l'oxalamate di(hydrogéné) de suif d'ammonium di(hydrogéné) de suif, le malonamate di(hydrogéné) de suif d'ammonium di(hydrogéné) de suif, le glutaramate di(hydrogéné) de suif d'ammonium di(hydrogéné) de suif, l'adipamate di(hydrogéné) de suif d'ammonium di(hydrogéné) de suif, le pimélamate di(hydrogéné) de suif d'ammonium di(hydrogéné) de suif, le subaramate di(hydrogéné) de suif d'ammonium di(hydrogéné) de suif.

6. Emulsion selon la revendication 5, comprenant en plus un acide de formule 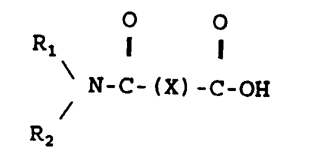 (dans laquelle R₁ et R₂ sont identiques ou différents et représentent un alkyle ou un alcényle ayant de 8 à 40 atomes de carbone, et X représente un phényle ortho, para, ou métasubstitué, ou un alkylène ou un alcénylène ayant de 1 à 6 atomes de carbone, ou un cycloalkylène ayant de 5 à 7 atomes de carbone) où la quantité combinée d'acide et de sel est d'environ 0,05 à environ 20%.

7. Emulsion selon la revendication 6, dans laquelle l'acide et le sel sont présents dans un rapport allant d'environ 99:1 à environ 1:99.

8. Emulsion selon la revendication 7, dans laquelle l'acide et le sel sont présents dans un rapport allant d'environ 70:30 à environ 30:70.

9. Emulsion selon la revendication 7, dans laquelle la quantité combinée d'acide et de sel est d'environ 0,05 à environ 20%.

10. Emulsion selon la revendication 9, dans laquelle la quantité combinée d'acide et de sel est d'environ 3 à 7%.

11. Emulsion selon la revendication 10, comprenant en plus une quantité démêlante efficace d'au moins un composé à base de silicone.

12. Composition de shampooing formulée caractérisée en ce qu'elle contient une quantité allant d'environ 0,05 à environ 20% d'un sel de formule : 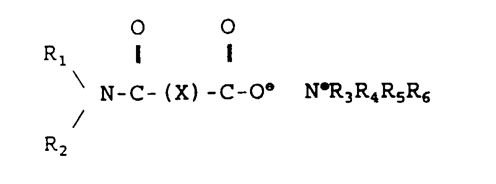 dans laquelle R₁ et R₂ sont identiques ou différents et représentent un alkyle ou un alcényle ayant de 8 à 40 atomes de carbone, R₃ et R₄ sont identiques à R₁ et R₂, et R₅ et R₆ sont un atome d'hydrogène ; ou R₃, R₄ , R₅ et R₆ sont, indépendamment, un atome d'hydrogène, un alkyle ou un alcényle ayant de 1 à 40 atomes de carbone quand R₁ et R₂ sont un alkyle ou un alcényle ayant de 14 à 22 atomes de carbone ; et X représente un phényle ortho, para, ou métasubstitué, ou un alkylène ou un alcénylène ayant de 1 à 6 atomes de carbone, ou un cycloalkylène ayant de 5 à 7 atomes de carbone.

13. Composition de shampooing selon la revendication 12, dans laquelle la quantité de sel est d'environ 0,05 à environ 20%.

14. Composition de shampooing selon la revendication 13, dans laquelle R₁, R₂, R₃ et R₄ sont des groupes alkyles gras de suif, et R₅ et R₆ sont un atome d'hydrogène.

15. Composition de shampooing selon la revendication 14, comprenant en plus une quantité démêlante efficace d'au moins un composé à base de silicone.

16. Composition de shampooing selon la revendication 13, comprenant en plus un acide de formule 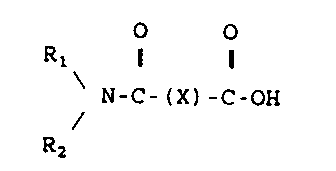 (dans laquelle R₁ et R₂ sont identiques ou différents et représentent un alkyle ou un alcényle ayant de 8 à 40 atomes de carbone, et X représente un phényle ortho, para, ou métasubstitué, ou un alkylène ou un alcénylène ayant de 1 à 6 atomes de carbone, ou un cycloalkylène ayant de 5 à 7 atomes de carbone) où la quantité combinée d'acide et de sel est d'environ 0,05 à environ 20%.

17. Composition de shampooing selon la revendication 16, dans laquelle l'acide et le sel sont présents dans un rapport allant d'environ 99:1 à environ 1:99.

18. Composition de shampooing selon la revendication 17, dans laquelle le sel est choisi dans le groupe formé par le succinamate di(hydrogéné) de suif d'ammonium di(hydrogéné) de suif, le cyclohexamate di(hydrogéné) de suif d'ammonium di(hydrogéné) de suif, l'isophtalamate di(hydrogéné) de suif d'ammonium di(hydrogéné) de suif, le maléamate di(hydrogéné) de suif d'ammonium di(hydrogéné) de suif, l'oxalamate di(hydrogéné) de suif d'ammonium di(hydrogéné) de suif, le malonamate di(hydrogéné) de suif d'ammonium di(hydrogéné) de suif, le glutaramate di(hydrogéné) de suif d'ammonium di(hydrogéné) de suif, l'adipamate di(hydrogéné) de suif d'ammonium di(hydrogéné) de suif, le pimélamate di(hydrogéné) de suif d'ammonium di(hydrogéné) de suif, le subaramate di(hydrogéné) de suif d'ammonium di(hydrogéné) de suif.

19. Suspension de particules dans un système liquide comprenant des particules qui sont insolubles dans le système liquide caractérisée en ce que ladite suspension contient une quantité de mise en suspension efficace d'un sel de formule : 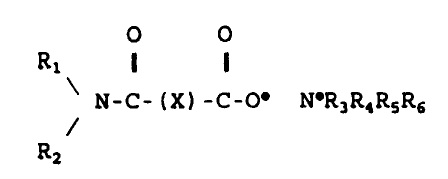 dans laquelle R₁ et R₂ sont identiques ou différents et représentent un alkyle ou un alcényle ayant de 8 à 40 atomes de carbone, R₃ et R₄ sont identiques à R₁ et R₂, et R₅ et R₆ sont un atome d'hydrogène ; ou R₃, R₄ , R₅ et R₆ sont, indépendamment, un atome d'hydrogène, un alkyle ou un alcényle ayant de 1 à 40 atomes de carbone quand R₁ et R₂ sont un alkyle ou un alcényle ayant de 14 à 22 atomes de carbone ; et X représente un phényle ortho, para, ou métasubstitué, ou un alkylène ou un alcénylène ayant de 1 à 6 atomes de carbone, ou un cycloalkylène ayant de 5 à 7 atomes de carbone.

20. Formulation d'antisudoral caractérisée en ce qu'elle contient une quantité dans la gamme d'environ 0,05 à environ 20% d'un sel de formule : 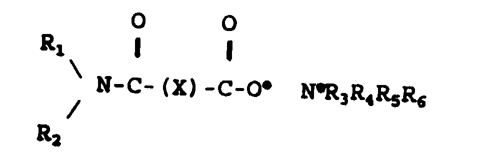 dans laquelle R₁ et R₂ sont identiques ou différents et représentent un alkyle ou un alcényle ayant de 8 à 40 atomes de carbone, R₃ et R₄ sont identiques à R₁ et R₂, et R₅ et R₆ sont un atome d'hydrogène ; ou R₃, R₄ , R₅ et R₆ sont, indépendamment, un atome d'hydrogène, un alkyle ou un alcényle ayant de 1 à 40 atomes de carbone quand R₁ et R₂ sont un alkyle ou un alcényle ayant de 14 à 22 atomes de carbone ; et X représente un phényle ortho, para, ou métasubstitué, ou un alkylène ou un alcénylène ayant de 1 à 6 atomes de carbone, ou un cycloalkylène ayant de 5 à 7 atomes de carbone.

21. Formulation selon la revendication 20, dans laquelle la quantité de sel va d'environ 0,05 à environ 20%.

22. Formulation selon la revendication 21, dans laquelle R₁, R₂, R₃ et R₄ sont des groupes alkyles gras de suif, et R₅ et R₆ sont un atome d'hydrogène.

23. Formulation selon la revendication 20, comprenant en plus un acide de formule 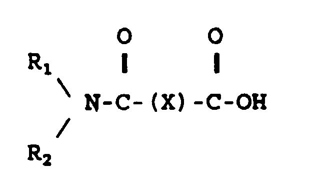 (dans laquelle R₁ et R₂ sont identiques ou différents et représentent un alkyle ou un alcényle ayant de 8 à 40 atomes de carbone, et X représente un phényle ortho, para, ou métasubstitué, ou un alkylène ou un alcénylène ayant de 1 à 6 atomes de carbone, ou un cycloalkylène ayant de 5 à 7 atomes de carbone) où la quantité combinée d'acide et de sel est d'environ 0,05 à environ 20%.
